(19) Europäisches Patentamt
European Patent Office
Office européen des brevets

(11) Publication number: **0 219 541 B1**

# (12) EUROPEAN PATENT SPECIFICATION

(45) Date of publication of patent specification: **20.09.89**

(51) Int. Cl.$^4$: **A 61 K 31/55**

(21) Application number: **86902985.0**

(22) Date of filing: **16.04.86**

(86) International application number: **PCT/US86/00808**

(87) International publication number: **WO 86/06631 20.11.86 Gazette 86/25**

## (54) TREATMENT OF PREMENSTRUAL SYNDROMES USING TRIAZOLOBENZODIAZEPINES.

(30) Priority: **03.05.85 US 730703**

(43) Date of publication of application: **29.04.87 Bulletin 87/18**

(45) Publication of the grant of the patent: **20.09.89 Bulletin 89/38**

(84) Designated Contracting States: **AT BE CH DE FR GB IT LI LU NL SE**

(56) References cited: **None**

(73) Proprietor: **THE UPJOHN COMPANY**
**7000 Portage Road**
**Kalamazoo, Michigan 49001 (US)**

(72) Inventor: **PYKE, Robert, E.**
**2212 Lloyd Center**
**Portland, OR 97232 (US)**

(74) Representative: **Perry, Robert Edward et al**
**GILL JENNINGS & EVERY 53-64 Chancery Lane**
**London WC2A 1HN (GB)**

**EP 0 219 541 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the invention

The dramatic changes in mood, behaviour, cognition, and somatic functioning in some women in relation to the menstrual cycle, the so-called premenstrual syndrome(s), have become the focus of a great deal of public and medical scrutiny. See Rubinow et al., Premenstrual Syndromes. Overview from a Methodologic Perspective, Am. J. Psychiatry, 141, 163—172, February, 1984; Heneson, The Selling of PMS, Science 84, 67—71, May, 1984; Rubinow et al., Prospective Assessment of Menstrually Related Mood Disorders, Am. J. Psychiatry 141, 684—686, May 1984.

At the 1983 PMS two-day workshop at the National Institute of Mental Health (NIHM), Rockville, MD., the operational definition of "premenstrual syndromes" finally arrived at: marked change in intensity of symptoms measured (daily) from cycle days 5 to 10, compared to the intensity within the six day interval prior to menses, for at least two consecutive cycles. See JAMA 249, 2866 (1983).

Premenstrual depressive symptoms are not usually those of classic endogenous depression (eq. psychomotor retardation, early morning awakening, loss of appetite and weight); although some investigators believe that premenstrual dysphoric changes might serve as models for certain nonendogenous forms of depression. See Premenstrual Syndromes, depression linked, JAMA, 249, 2864—2866 (1983) and Halbreich et al., Premenstrual Depressive Changes, Arch. Gen. Psychiatry, 40, 535—542 (1983).

Harrison et al reported in Treatment of Premenstrual Dysphoric Changes: Clinical Outcome and Methodological Implications, Psychopharmacology Bulletin, 20, 118—122 (1984):

"Treatment studies of premenstrual changes have been disappointing. Treatments which have been tried include progesterone, lithium, bromocriptine, progestagens, pyridoxine, diuretics, benzodiazepines, and prostaglandin synthetase inhibitors. While uncontrolled studies with a wide variety of agents have yielded favorable results, placebo-controlled trials have generally either failed to show significant drug-placebo differences or even found placebo to be the most effective treatment (Green, 1982). As in studies of psychosomatic disorders, the placebo response in premenstrual changes shows very wide range — from 30 to 80%. This has led to a pessimistic attitude about the possibility of demonstrating drug efficacy in this condition, and there has been a decline of interest in conducting premenstrual changes treatment studies."

Alprazolam, 8-chloro-1-methyl-6-phenyl-4H-s-triazolo[4,3-a][1,4]-benzodiazepine, is a triazolobenzodiazepine that has been reported to have both anxiolytic and anti-depressive effects. See Feighner, Benzodiazepines as Antidepressants, Mod. Prob. Pharmacopsychiat. *18*; 197—213 (1982).

Xanax$^R$ Tablets are manufactured by The Upjohn Company and contain alprazolam. Xanax Tablets are available in the USA, upon prescription, in 0.25, 0.5 and 1.0 mg strengths.

Adinazolam, 8-chloro-1[(dimethylamino)methyl]-6-phenyl-4H-s-triazolo[4,3-a][1,4]benzodiazepine, and other 1-(nitrogen-containing group)-triazolobenzodiazepines and their pharmaceutically-acceptable acid addition salts, are also reported to exhibit anti-depressive activities. See US—A—4264615 and US—A—4012413. $N_5$-oxides of such triazolobenzodiazepines are disclosed in US—A—4235775 and DE—A—2201210, $N_1$-oxides are disclosed in US—A—4009175.

Summary of the invention

According to the present invention, a compound of formula I or a $N_5$-oxide, $N_1$-oxide (when n=1), hydrate, solvate or pharmacologically-acceptable acid addition salt thereof, is used for the preparation of a medicament for use in the prevention or treatment of a woman's menstrually-related mood disorder, by administration for 1 to 10 days prior to menses. In formula I (see below), n is 1 or 2 and $R_1$ and $R_2$ are independently selected from hydrogen and $C_{1-3}$ alkyl (i.e. methyl, ethyl, propyl or isopropyl).

Description of the invention

Compounds for use in this invention can be prepared by methods disclosed in US—A—3842090, US—A—3947466, US—A—3957761 US—A—4001262, US—A—4012413, US—A—4075221, US—A—4250094, US—A—4684725 and EP—A—0146306.

The $N_5$-oxides of the compounds of the formula I compounds are prepared from the known 7-chloro-2-hydrazino-5-phenyl-3H-1,4-benzodiazepine, 4-oxide (compound g of Table I of K. Meguro et al, Chem. Pharm. Bull., 21(11), 2382—2390 (1973)). The $N_5$-oxides of compounds herein wherein n is 1 are prepared using said 4-oxide in the methods described in US—A—4235775. The $N_5$-oxides of compounds herein wherein n is 2 are prepared using said 4-oxide in the methods described in Scheme B in US—A—4012413. The oxides of the nitrogen contained in the 1-position substituent of Formula I compounds wherein n is 1 are prepared as described in US—A—4003175.

Acid addition salts of compounds of the Formula I can be prepared by neutralization of the free base with the appropriate amount of an inorganic or organic acid, examples of which are hydrochloric, hyrobromic, sulfuric, nitric, phosphoric, acetic, lactic, benzoic, salicyclic, glycolic, succinic, tartaric, maleic, malic, pamoic, cyclohexanesulfamic, citric and methanesulfonic acids, and the like acids. The neutralization can be carried out by a variety of procedures known to the art to be generally useful for the preparation of amine acid addition salts as well as the procedure(s) disclosed in US—A—426415 and US—A—4012413. These acid addition salts are useful for upgrading the free bases and an equivalent amount of the

pharmacologically acceptable salts can be used in the same manner as the free base.

For oral administration either solid or fluid unit dosage forms can be prepared. For preparing solid compositions such as tablets, the compound of Formula I is mixed with conventional ingredients such as talc, magnesium stearate, dicalcium phosphate, magnesium aluminum silicate, calcium sulfate, starch, lactose, acacia, methylcellulose and functionally similar materials as pharmaceutical diluents or carriers. Wafers are prepared in the same manner as tablets, differing only in shape and the inclusion of sucrose or other sweetner and flavor. In their simplest embodiment, capsules, like tablets, are prepared by mixing the compound with an inert pharmaceutical diluent and filling the mixture into a hard gelatin capsule of appropriate size. Soft gelatin capsules are prepared by machine encapsulation of a slurry of the compound with an acceptable vegetable oil, light liquid petrolatum or other inert oil.

Fluid unit dosage forms for oral administration such as syrups, elixirs, and suspensions can be prepared. The water-soluble forms can be dissolved in an equeous vehicle together with sugar, aromatic flavoring agents and preservatives to form a syrup. An elixir is prepared by using a hydro-alcoholic (ethanol) vehicle with suitable sweeteners such as sugar and saccharin, together with an aromatic flavoring agent.

Suspensions can be prepared with a syrup vehicle with the aid of a suspending agent such as acacia, tragacanth, methylcellulose and the like.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilized before filling into a suitable vial or ampul and sealing. Advantageously, adjuvants such as a local anesthetic, preservative and buffering agents can be dissolved in the vehicle. To enhance the stability, the composition can be frozen after filling into the vial and the water removed under vacuum. The dry lyophilized powder is then sealed in the vial and an accompanying vial or water for injection is supplied to reconstitute the liquid prior to use. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions of the present invention are presented for administration to humans in unit dosage forms, such as tablets, capsules, pills, powders, granules, sterile parenteral solutions or suspensions, and oral solutions or suspensions, and oil in water and water in oil emulsions containing suitable quantities of the compound of Formula I. The term unit dosage form as used in this specification and claims refers to physically discrete units suitable as unitary dosages for human subjects and animals, each unit containing a predetermined quantity of an active material calculated to prevent/treat the menstrually related mood disorder in asociation with the required pharmaceutical diluent, carrier or vehicle. The specifications for the novel unit dosage forms of this invention are dictated by and directly dependent on (a) the unique characteristics of the active material and the particular effect to be achieved, and (b) the limitations inherent in the art of compounding such an active material for use in humans and animals, as disclosed in detail in this specification, these being features of the present invention. Examples of suitable unit dosage forms in accord with this invention are tablets, capsules, pills, suppositories, powder packets, granules, waters, cachets, teaspoonfuls, tablespoonfuls, dropperfuls, ampuls, vials, segregated multiples of any of the foregoing, and other forms as herein described.

The dosage of the compound for treatment depends on route of administration, the age, weight, and condition of the patient and the particular compound.

When n is 2, a dosage schedule of from about 10 to about 200 mg/day in a single or divided dose, embraces the effective range for preventing or treating menstrually related mood disorders. The dosage to be administered is calculated on the basis of from about 0.1 to about 3 mg/kg by body weight of subject per day. Generally, depending upon the conditions of concern to the prescribing physician, a dosage schedule of from about 10 to about 200 mg/day in divided doses, embraces the effective range of 8-chloro-1-[2-(dimethylamino)ethyl]-6-phenyl-4H-s-triazolo[4,3-a][1,4]benzodiazepine p-toluenesulfonate (1:1), for preventing and/or treating menstrually related mood disorders.

When n is 1, a dosage schedule of from about 10 to about 120 mg/day in a single or divided dose, embraces the effective range for preventing or treating menstrually related mood disorders. The dosage to be administered is calculated on the basis of from about 0.1 to about 2 mg/kg by body weight of subject per day. Generally, depending upon the conditions of concern to the prescribing physician, a dosage schedule of from about 10 to about 120 mg/day in divided doses, preferably about 40 to about 100 mg/day in divided doses, embraces the effective range of adinazolam, 8-chloro-1-[(dimethylamino)methyl]-6-phenyl-4H-s-triazolo[4,3-a][1,4]benzodiazepine, for preventing and/or treating menstrually related mood disorders.

The compounds of Formula I are compounded with a suitable pharmaceutical carrier in unit dosage form for convenient and effective administration. In the preferred embodiment of this invention for adinazolam, the dosage units contain the compound as the mesylate salt (nonhygroscopic) in: 5, 10, 20, and 30 mg amounts for systemic treatment.

The compositions are useful in preventing and treating menstrually related mood disorders (including a premenstrual depression syndrome, a premenstrual affective syndrome, a premenstrual cognitive

syndrome, a premenstrual neurovegetative syndrome and a premenstrual behavioral syndrome) upon administration immediately preceding and/or during the symptomatic portion of the menstrual cycle. In general, the compositions are administered for 1 to 10 days prior to menses. The dosage is then stopped, preferably by tapering the daily dose downward and then stopping the dosing during the menses.

It is, of course, well-recognized that the daily dose can be formulated in a sustained release tablet or other dosage form, utilizing, for example, hydroxypropylmethyl cellulose or other cellulose ethers as disclosed in US—A—3 065 143 if desired, to provide for a once or twice a day administration schedule.

The term "menstrually related mood disorder" as used in the subject specification and claims means a marked change in intensity of symptoms measured (daily) during an intermenstrual baseline period (e.g., from cycle days 5 to 10), compared with the intensity of these symptoms during the symptomatic portion of the menstrual cycle, (e.g., during the six day interval prior to menses), for at least two consecutive cycles.

The term "premenstrual depression syndrome" as used in the subject specification and claims means a marked change in intensity of symptoms measured (daily) during an intermenstrual baseline period (e.g., from cycle days 5 to 10), compared with the intensity of these symptoms during the symptomatic portion of the menstrual cycle, (e.g., during the six day interval prior to menses), for at least two consecutive cycles, and meeting the criteria for PAF depression syndrome given in Table 1, Premenstrual Depressive Changes, Arch. Gen. Psychiatry, 40, 535—542, 536 (1983).

The common symptoms of other premenstrual (affective, cognitive, neurovegetative and behavioral) syndromes are described in Appendix I, Premenstrual Syndromes: Overview from a Methodologic Perspective, Am. J. Psychiatry 141:2, 163—172 170 (1984).

In a double-blind 28-day/cross-over clinical trial for the evaluation of panic disorders, the effects of alprazolam (1 to 6 mg/day) and adinazolam mesylate (20 to 120 mg/day) on menstrually related mood disorders were noted in eight female subjects under the age of fifty years:

Histories of premenstrual disorder were available from the medical record for four of these subjects; the others were new to the clinic at the start of the study and did not complain of premenstrual disorder or had simply not reported a premenstrual disorder previously though they had been seen as clinic patients before the study.

A single-blind placebo was given the seven days as the first treatment in the study.

The alprazolam tablets or adinazolam mesylate tablets were given double-blind for twenty-eight days. Finally, each subject was crossed over double-blind to the other respective active drug for another twenty-eight days.

Symptoms of irritability, loss of temper, or hostility were present in one subject during placebo use. With alprazolam, four subjects each reported such symptoms, each for less than two weeks; with adinazolam mesylate, three subjects each reported such symptoms, each for less than two weeks also. One other symptom apparently relevant to the premenstruum was reported, premenstrual pelvic pain. It was reported by one subject, with alprazolam only. However, the patient added that this symptom was a usual one of premenstrual syndrome for her, i.e., before the study. One woman had none of the above symptoms with any study drug.

In each of the seven cases with apparent premenstrual symptoms, the symptoms varied with the active drug used. In four cases, episodic irritability and the like were lesser with adinazolam mesylate than with alprazolam, i.e., absent compared to moderate, respectively, or mild compared to moderate, respectively. In two cases, such symptoms were absent with alprazolam but mild or severe with adinazolam mesylate. In the case of premenstrual pelvic pain, adinazolam mesylate prevented the pain completely, but alprazolam had no effect.

In terms of those with a known history of premenstrual irritability, two improved with adinazolam mesylate and not with alprazolam, and one improved with alprazolam and not with adinazolam mesylate.

In terms of those with any known history of premenstrual symptoms, three improved with adinazolam mesylate and not with alprazolam, and one improved with alprazolam and not with adinazolam mesylate.

In terms of those with a known or a presumptive history of pre-menstrual symptoms, five improved with adinazolam mesylate but not alprazolam, and two improved with alprazolam but not adinazolam mesylate.

Harrison et al reported in "Treatment of Premenstrual Syndromes", General Hospital Psychiatry, 7, 54—65, 62 (Jan. 1985):

"A new triazolobenzodiazepine, alprazolam, has been reported to have both anxiolytic and antidepressant effects [63]. There have been no controlled studies evaluating the use of alprazolam in the treatment of premenstrual symptoms. We have had encouraging results with the use of alprazolam for treatment of premenstrual affective changes in a small pilot study and are in the process of conducting a placebo-controlled trial in those women whose predominant complaints include insomnia, anxiety, tension, and irritability. The dose of alprazolam must be titrated on an individual basis, determined by the patient's susceptibility to the major side effect, that is, daytime sedation. Our experience suggests a daily dose range of 1 mg—4 mg, with two-thirds of the dose being given at bedtime. Treatment should be restricted to the symptomatic portion of the premenstrual cycle. Tapering off the dose during the first three days of the menses is advisable to prevent the possibility of withdrawal seizures when high doses are used".

The use of alprazolam to prevent or treat a menstrually related mood disorder is the invention of

another and claimed in a U.S. Patent Application, Serial No. 730,702, filed May 3, 1985.

The following examples are illustrative of the best mode contemplated by the inventor for carrying out his invention and are not to be construed as limiting.

Example 1

A lot of 1,000 tablets, each containing 20 mg of 8-chloro-1-[(di-methylamino)methyl]-6-phenyl-4H-S-triazolo[4,3-α][1,4]-benzodiazepine methanesulfonate, adinazolam mesylate, is prepared from the following types and amounts of ingredients:

| | |
|---|---|
| Adinazolam mesylate, nonhygroscopic | 20 g |
| Lactose, USP (hydrose Spray Standard) | 245 g |
| Collodial Silicon Dioxide, NF | 5 g |
| Corn Starch, NF (Bolted) | 15.3 g |
| Croscarmellose Sodium, NF (Type A) | 9.6 g |
| Microcrystalline Cellulose, NF (Medium Powder) | 49 g |
| Magnesium Stearate, NF (Powder) | 2.1 g |

The ingredients are mixed well for up to two minutes in a high shear mixer and compressed into scored tablets.

The tablets are useful in treating menstrually related mood disorders in a female at a dose of ½ to 1 tablet every 6 hours for 5—7 days prior to menses. At the onset of menses, the daily dose is tapered and stopped over a 3—5 day period.

Example 2

A lot of 1,000 tablets, each containing 10 mg of 8-chloro-1-[(dimethylamino)methyl]-6-phenyl-4H-s-triazolo[4,3-a][1,4]benzodiazepine methanesulfonate, adinazolam mesylate, is prepared from the following types and amounts of ingredients:

| | |
|---|---|
| Adinazolam mesylate, nonhygroscopic | 10 g |
| Dicalcium phosphate | 1500 g |
| Methylcellulose, U.S.P. (15 cps) | 60 g |
| Talc | 150 g |
| Corn Starch, NF | 200 g |
| Calcium stearate | 12 g |

The compound and dicalcium phosphate are mixed well, granulated with 7.5 percent solution of methylcellulose in water, passed through a No. 8 screen and dried carefully. The dried granules are passed through a No. 12 screen, mixed thoroughly with the talc, starch and stearate, and compressed into tablets.

The tablets are useful in treating menstrually related mood disorders in a human female at a dose of 1 or 2 tablet(s) every 6 hours for 7 days prior to menses. At the onset of menses, the daily dose is tapered and stopped over a 3—5 day period.

Example 3

One thousand two-piece hard gelatin capsules, each containing 10 mg of 8-chloro-1[(dimethylamino)-methyl]-6-phenyl-4H-s-triazolo[4,3-a][1,4]benzodiazepine methanesulfonate, adinazolam mesylate, are prepared from the following types and amounts of ingredients:

| | |
|---|---|
| Adinazolam mesylate, nonhygroscopic | 10 g |
| Talc | 25 g |
| Magnesium stearate, NF (powdered) | 250 g |

The ingredients are mixed well and filled into capsules of the proper size.

Capsules so prepared are useful to treat premenstrual depression syndrome in a human female at a

dose of 1 or 2 capsule(s) every 4 hours for 7 days prior to menses. At the onset of menses, the daily dose is tapered and stopped over a 3—5 day period.

Example 4

Following the procedures of the preceding Examples 1—4, unit dosage forms are similarly prepared by substituting for adinazolam mesylate an equal molar amount of:

8-chloro-1-[(dimethylamino)methyl]-6-phenyl-4H-s-triazolo[4,3-a][1,4]benzodiazepine,

8-chloro-1-[(dimethylamino)methyl]-6-phenyl-4H-s-triazolo[4,3-a][1,4]benzodiazepine, methanesulfonate hydrate,

1-(aminomethyl)-8-chloro-6-phenyl-4H-s-triazolo[4,3-a][1,4]benzodiazepine,

8-chloro-1-[(methylamino)methyl]-6-phenyl-4H-s-triazolo[4,3-a][1,4]benzodiazepine,

8-chloro-1-[2-(dimethylamino)ethyl]-6-phenyl-4H-s-triazolo[4,3-a][1,4]benzodiazepine,

8-chloro-1-[2-(dimethylamino)ethyl]-6-phenyl-4H-s-triazolo[4,3-a][1,4]benzodiazepine, p-toluenesulfonate (1:1),

8-chloro-1-[2-(dimethylamino)ethyl]-6-phenyl-4H-s-triazolo[4,3-a][1,4]benzodiazepine, cyclohexanesulfamate (1:2),

8-chloro-1-[2-(dimethylamino)methyl]-6-phenyl-4H-s-triazolo[4,3-a][1,4]benzodiazepine, 5-oxide and

8-chloro-1-[2-(dimethylamino)methyl]-6-phenyl-4H-s-triazolo[4,3-a][1,4]benzodiazepine, $N^1$-oxide.

FORMULA

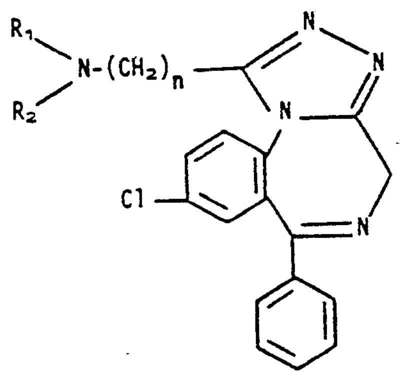

I

**Claims**

1. Use of a compound for the preparation of a medicament for use in the prevention or treatment of a woman's menstrually-related mood disorder, by administration for 1 to 10 days prior to menses, in which the compound has the formula

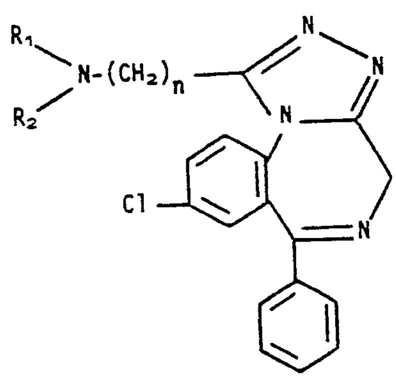

I

wherein n is 1 or 2 and $R_1$ and $R_2$ are independently selected from hydrogen and $C_1$—$C_3$ alkyl, or a pharmacologically-acceptable acid addition salt, hydrate, $N_5$-oxide or $N_1$-oxide (when n is 1) thereof.

2. Use according to claim 1, wherein the compound is 8-chloro-1-[(dimethylamino)methyl]-6-phenyl-4H-s-triazolo[4,3-a][1,4]benzodiazepine.

3. Use according to claim 1, wherein the compound is 8-chloro-1-[(dimethylamino)methyl]-6-phenyl-4H-s-triazolo[4,3-a][1,4]benzodiazepine methanesulfonate or the hydrate thereof.

4. Use according to claim 1, wherein the compound is 1-(aminomethyl)-8-chloro-6-phenyl-4H-s-triazolo[4,3-a][1,4]benzodiazepine.

5. Use according to claim 1, wherein the compound is 8-chloro-1-[(methylamino)methyl]-6-phenyl-4H-s-triazolo[4,3-a][1,4]benzodiazepine.

6. Use according to claim 1, wherein the compound is 8-chloro-1-[2-(dimethylamino)ethyl]-6-phenyl-4H-s-triazolo[4,3-a][1,4]benzodiazepine p-toluenesulfonate (1:1).

7. Use according to claim 1, wherein the compound is 8-chloro-1-[2-(dimethylamino)ethyl]-6-phenyl-4H-s-triazolo[4,3-a][1,4]benzodiazepine cyclohexanesulfamate (1:2).

8. Use according to claim 1, wherein the compound is 8-chloro-1-[2-(dimethylamino)ethyl]-6-phenyl-4H-s-triazolo[4,3-a][1,4]benzodiazepine.

9. Use according to any preceding claim, wherein the menstrually-related mood disorder is selected from a premenstrual depression syndrome, a premenstrual affective syndrome, a premenstrual cognitive syndrome, a premenstrual neurovegetative syndrome or a premenstrual behavioural syndrome.

10. Use according to claim 9, wherein the disorder is a premenstrual depression syndrome.

**Patentansprüche**

1. Verwendung einer Verbindung zur Herstellung eines Pharmazeutikums zur Anwendung in der Vorbeugung oder Behandlung von bei Frauen auftretenden menstruell bedingten Stimmungsschwankungen durch Verabreichung eines Pharmazeutikums während 1 bis 10 Tagen vor der Menstruation, in welchem die Verbindung folgende Formel aufweist

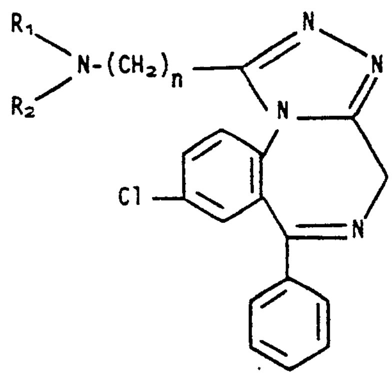

I

worin n 1 oder 2 ist und $R_1$ und $R_2$ unabhängig voneinander aus Wasserstoff und $C_1$—$C_3$ Alkyl ausgewählt werden, oder ein pharmakologisch verträgliches Säureadditionssalz, Hydrat, $N_5$-Oxid oder $N_1$-Oxid (wenn n = 1) davon.

2. Verwendung nach Anspruch 1, worin die Verbindung 8-Chlor-1-[(dimethylamino)methyl]-6-phenyl-4H-s-triazolo[4,3-a][1,4]benzodiazepin ist.

3. Verwendung nach Anspruch 1, worin die Verbindung 8-Chlor-1-[(dimethylamino)methyl]-6-phenyl-4H-s-triazolo[4,3-a][1,4]benzodiazepin Methansulfonat oder das Hydrat davon ist.

4. Verwendung nach Anspruch 1, worin die Verbindung 1-(Aminomethyl)-8-chlor-6-phenyl-4H-s-triazolo[4,3-a][1,4]benzodiazepin ist.

5. Verwendung nach Anspruch 1, worin die Verbindung 8-Chlor-1-[(methylamino)methyl]-6-phenyl-4H-s-triazolo[4,3-a][1,4]benzodiazepin ist.

6. Verwendung nach Anspruch 1, worin die Verbindung 8-Chlor-1-[2-(dimethylamino)ethyl]-6-phenyl-4H-s-triazolo[4,3-a][1,4]benzodiazepin p-Toluolsulfonat (1:1) ist.

7. Verwendung nach Anspruch 1, worin die Verbindung 8-Chlor-1-[2-(dimethylamino)ethyl]-6-phenyl-4H-s-triazolo[4,3-a][1,4]benzodiazepin Cyclohexansulfamat (1:2) ist.

8. Verwendung nach Anspruch 1, worin die Verbindung 8-Chlor-1-[2-(dimethylamino)ethyl]-6-phenyl-4H-s-triazolo[4,3-a][1,4]benzodiazepin ist.

9. Verwendung nach einem der vorhergehenden Ansprüche, worin die bei Frauen auftretenden menstruell bedingten Stimmungsschwankungen aus einer vormenstruellen Depression, einem vormenstruellen manisch-depressiven Syndrom, einem vormenstruellen Erkenntnissyndrom, einem vormenstruellen neuronegativen Syndrom oder einer vormenstruellen Verhaltensstörung gewählt sind.

10. Verwendung nach Anspruch 9, worin die Störung eine vormenstruelle Depression ist.

**Revendications**

1. Utilisation d'un composé pour la préparation d'un médicament destiné à être utilisé dans la prévention ou le traitement d'un trouble de l'humeur lié aux règles chez la femme, par administration pendant 1 à 10 jours avant les règles, dans laquelle le composé répond à la formule

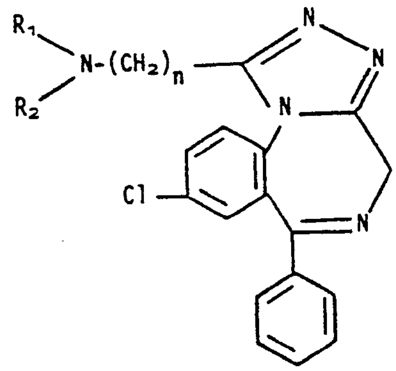

I

dans laquelle n est égal à 1 ou 2 et $R_1$ et $R_2$ sont choisis indépendamment entre l'hydrogène et un groupe alkyle en $C_1$ à $C_3$, ou bien d'un sel d'addition d'acide pharmacologiquement acceptable, d'un hydrate, d'un $N_5$-oxyde ou d'un $N_1$-oxyde (lorsque n est égal à 1) de ce composé.

2. Utilisation suivant la revendication 1, dans laquelle le composé est la 8-chloro-1-[(diméthylamino)-méthyl]-6-phényl-4H-s-triazolo[4,3-a][1,4]benzodiazépine.

3. Utilisation suivant la revendication 1, dans laquelle le composé est le méthanesulfonate de 8-chloro-1-[(diméthylamino)méthyl]-6-phényl-4H-s-triazolo[4,3-a][1,4]benzodiazépine ou son hydrate.

4. Utilisation suivant la revendication 1, dans laquelle le composé est la 1-(aminométhyl)-8-chloro-6-phényl-4H-s-triazolo[4,3-a][1,4]benzodiazépine.

5. Utilisation suivant la revendication 1, dans laquelle le composé est la 8-chloro-1-[(méthylamino)-méthyl]-6-phényl-4H-s-triazolo[4,3-a][1,4]benzodiazépine.

6. Utilisation suivant la revendication 1, dans laquelle le composé est le p-toluènesulfonate de 8-chloro-1-[2-(diméthylamino)éthyl]-6-phényl-4H-s-triazolo[4,3-a][1,4]benzodiazépine (1:1).

7. Utilisation suivant la revendication 1, dans laquelle le composé est le cyclohexanesulfamate de 8-chloro-1-[2-(diméthylamino)éthyl]-6-phényl-4H-s-triazolo[4,3-a][1,4]benzodiazépine (1:2).

8. Utilisation suivant la revendication 1, dans laquelle le composé est la 8-chloro-1-[2-(diméthylamino)éthyl]-6-phényl-4H-s-triazolo[4,3-a][1,4]benzodiazépine.

9. Utilisation suivant l'une quelconque des revendications précédentes, dans laquelle le trouble de l'humeur lié aux règles est un syndrome dépressif prémenstruel, un syndrome affectif prémenstruel, un syndrome cognitif prémenstruel, un syndrome neurovégétatif prémenstruel ou bien un syndrome comportemental prémenstruel.

10. Utilisation suivant la revendication 9, dans laquelle le trouble est un syndrome dépressif prémenstruel.